# EUROPEAN PATENT APPLICATION

(11) **EP 3 067 780 A1**
(43) Date of publication of application: **14.09.2016**
(21) Application number: 13898559.3
(22) Date of filing: 06.12.2013
(51) Int. Cl.: G06F 3/01

(54) **METHOD FOR CONTROLLING TERMINAL DEVICE, AND WEARABLE ELECTRONIC DEVICE**

(71) Applicant: Huawei Device Co., Ltd., Shenzhen, Guangdong 518129 (CN)
(72) Inventor: TONG, Shuang, Shenzhen Guangdong 518129 (CN); LI, Shan, Shenzhen Guangdong 518129 (CN); YAO, Chaoqun, Shenzhen Guangdong 518129 (CN)
(74) Representative: Pfenning, Meinig & Partner mbB
(86) International application number: PCT/CN2013/088764
(87) International publication number: WO 2015/081558

(57) **Abstract**

Embodiments of the present invention disclose a method for controlling a terminal device, and a wearable electronic device, which are used to intelligently control another device by using the wearable electronic device. The method in the embodiments of the present invention includes: collecting, by the wearable electronic device, indicator data of a user's body; determining whether the indicator data meets a preset trigger condition; and if the indicator data meets the preset condition, sending a control command to the terminal device, so that the terminal device performs a preset operation.

## Description

### TECHNICAL FIELD

Embodiments of the present invention relate to the data processing field, and in particular, to a method for controlling a terminal device, and a wearable electronic device.

### BACKGROUND

With rapid development of science and technologies, a wearable electronic device is now gradually coming into our life. From sunglasses used for cheating in gambling in the earliest to current Google Glass, a Samsung Gear smartwatch, Jawbone UP, a ring that can be used as a mouse, and the like, these intelligent wearable electronic devices can help us handle life affairs more conveniently.

Product appearance forms of wearable electronic devices include a bracelet, a watch, glasses, a waist-hung device, and even a body-implanted device, and there are more such body-combined intelligent devices. By using a wearable electronic device, more help is provided for us faster in fields such as sports, medical treatment, and safety monitoring. Many related technologies are becoming more mature. For example, a wearable electronic device for detecting a motion tracks and quantizes a step quantity, a distance, and calories consumption of a user mainly by using a three-axis gravity accelerator or a precise motion sensor, and a powerful algorithm; a wearable electronic device for detecting sleep detects a motion of a user by using an actigraphy, so as to determine that the user is awake, or in shallow sleep or deep sleep.

However, a current wearable electronic device mainly focuses on sports and health monitoring, and an intelligent information assistant, and can only collect data for analysis and display to a user, so that the user makes a decision, or controls the wearable electronic device itself according to the collected data, which limits an affair-handling capability of the wearable electronic device.

### SUMMARY

Embodiments of the present invention provide a method for controlling a terminal device, and a wearable electronic device, which are used to intelligently control the terminal device by using the wearable electronic device.

A first aspect of the embodiments of the present invention provides a method for controlling a terminal device, including:
collecting, by a wearable electronic device, indicator data of a user's body;
determining, by the wearable electronic device, whether the indicator data meets a preset trigger condition; and
if the indicator data meets the preset trigger condition, sending, by the wearable electronic device, a control command to the terminal device.

With reference to the first aspect of the embodiments of the present invention, in a first implementation manner of the first aspect of the embodiments of the present invention, before the sending, by the wearable electronic device, a control command to the terminal device, the method includes:
determining, by the wearable electronic device, whether an automatic control function is enabled; and
if it is determined that the automatic control function is enabled, performing, by the wearable electronic device, the step of sending, by the wearable electronic device, a control command to the terminal device.

With reference to the first aspect and the first implementation manner of the first aspect of the embodiments of the present invention, in a second implementation manner of the first aspect of the embodiments of the present invention, the determining, by the wearable electronic device, whether the indicator data meets a preset trigger condition includes:
determining, by the wearable electronic device, whether the indicator data conforms to a sleep mode, where if the indicator data conforms to the sleep mode, it is determined that the indicator data meets the preset trigger condition, where the sleep mode is used to indicate that the user is in a sleep state;
   and/or
determining, by the wearable electronic device, whether the indicator data conforms to an awake mode, where if the indicator data conforms to the awake mode, it is determined that the indicator data meets the preset trigger condition, where the awake mode is used to indicate that the user is in an awake state.

With reference to the second implementation manner of the first aspect of the embodiments of the present invention, in a third implementation manner of the first aspect of the embodiments of the present invention,
the indicator data includes:
a quantity, of times of user motions, recorded by an actigraphy in the wearable electronic device;
the determining, by the wearable electronic device, whether the indicator data conforms to a sleep mode includes:
   if the quantity of times of user motions is less than a preset quantity of times, determining, by the wearable electronic device, that the indicator data conforms to the sleep mode; and
   the determining, by the wearable electronic device, whether the indicator data conforms to an awake mode includes:
      if the quantity of times of user motions is greater than or equal to the preset quantity of times, determining, by the wearable electronic device, that the indicator data conforms to the awake mode.

With reference to the second implementation manner of the first aspect of the embodiments of the present invention, in a fourth implementation manner of the first aspect of the embodiments of the present invention,
the indicator data includes:
a range, of a user motion, recorded by an actigraphy in the wearable electronic device;
the determining, by the wearable electronic device, whether the indicator data conforms to a sleep mode includes:
   if the range of the user motion is less than a preset range, determining, by the wearable electronic device, that the indicator data conforms to the sleep mode; and
   the determining, by the wearable electronic device, whether the indicator data conforms to an awake mode includes:
      if the range of the user motion is greater than or equal to the preset range, determining, by the wearable electronic device, that the indicator data conforms to the awake mode.

A second aspect of the embodiments of the present invention provides a wearable electronic device, including:
a sensing module, configured to collect indicator data of a user's body;
a condition determining module, configured to determine whether the indicator data collected by the sensing module meets a preset trigger condition; and
a transmission module, configured to: when the condition determining module determines that the indicator data meets the preset trigger condition, send a control command to a terminal device.

With reference to the second aspect of the embodiments of the present invention, in a first implementation manner of the second aspect of the embodiments of the present invention,
the device further includes:
an enabling determining module, configured to: when the condition determining module determines that the indicator data meets the preset trigger condition, determine whether an automatic control function is enabled; and
the transmission module is specifically configured to: when the enabling determining module determines that the automatic control function is enabled, send the control command to the terminal device.

With reference to the first implementation manner of the second aspect of the embodiments of the present invention, in a second implementation manner of the second aspect of the embodiments of the present invention, the condition determining module specifically includes:
a sleep triggering unit, configured to determine whether the indicator data collected by the sensing module conforms to a sleep mode, where if the indicator data conforms to the sleep mode, it is determined that the indicator data meets the preset trigger condition, where the sleep mode is used to indicate that the user is in a sleep state;
   and/or
an awake triggering unit, configured to determine whether the indicator data collected by the sensing module conforms to an awake mode, where if the indicator data conforms to the awake mode, it is determined that the indicator data meets the preset trigger condition, where the awake mode is used to indicate that the user is in an awake state.

A third aspect of the embodiments of the present invention provides a wearable electronic device, including:
a memory, a central processing unit, a peripheral interface, an RF circuit, a power management integrated circuit, an input/output subsystem, another input/control device, an external port, and a communications bus; where
the central processing unit performs the following operations:
   collecting indicator data of a user's body;
   determining whether the indicator data meets a preset trigger condition; and
   when the indicator data meets the preset trigger condition, sending a control command to a terminal device.

With reference to the third aspect of the embodiments of the present invention, in a first implementation manner of the third aspect of the embodiments of the present invention, the central processing unit specifically performs the following operations:
when the indicator data meets the preset trigger condition, determining whether an automatic control function is enabled; and
when it is determined that the automatic control function is enabled, triggering to perform the step of sending a control command to a terminal device.

With reference to the first implementation manner of the third aspect of the embodiments of the present invention, in a second implementation manner of the third aspect of the embodiments of the present invention, the central processing unit specifically performs the following operations:
determining whether the indicator data conforms to a sleep mode, where if the indicator data conforms to the sleep mode, it is determined that the indicator data meets the preset trigger condition, where the sleep mode is used to indicate that the user is in a sleep state;
   and/or
determining whether the indicator data conforms to an awake mode, where if the indicator data conforms to the awake mode, it is determined that the indicator data meets the preset trigger condition, where the awake mode is used to indicate that the user is in an awake state.

It may be learnt from the foregoing technical solutions that, the embodiments of the present invention have the following advantages: In the embodiments of the present invention, a wearable electronic device collects indicator data of a user's body, and when the indicator data meets a preset trigger condition, sends a control command, so that a terminal device performs a preset operation. In this way, the control command can be intelligently sent according to the indicator data collected by the wearable electronic device, so as to control the terminal device, which reduces operations of the user, and greatly improves an affair-handling capability of the wearable electronic device.

### BRIEF DESCRIPTION OF DRAWINGS

FIG. 1 is a schematic flowchart of a method for controlling a terminal device according to an embodiment of the present invention;
FIG. 2 is another schematic flowchart of a method for controlling a terminal device according to an embodiment of the present invention;
FIG. 3 is another schematic flowchart of a method for controlling a terminal device according to an embodiment of the present invention;
FIG. 4 is another schematic flowchart of a method for controlling a terminal device according to an embodiment of the present invention;
FIG. 5 is a schematic structural diagram of a wearable electronic device according to an embodiment of the present invention;
FIG. 6 is another schematic structural diagram of a wearable electronic device according to an embodiment of the present invention;
FIG. 7 is another schematic structural diagram of a wearable electronic device according to an embodiment of the present invention;
FIG. 8 is another schematic structural diagram of a wearable electronic device according to an embodiment of the present invention; and
FIG. 9 is another schematic structural diagram of a wearable electronic device according to an embodiment of the present invention.

### DESCRIPTION OF EMBODIMENTS

The following clearly and completely describes the technical solutions in the embodiments of the present invention with reference to the accompanying drawings in the embodiments of the present invention. Apparently, the described embodiments are merely some but not all of the embodiments of the present invention. All other embodiments obtained by a person skilled in the art based on the embodiments of the present invention without creative efforts shall fall within the protection scope of the present invention.

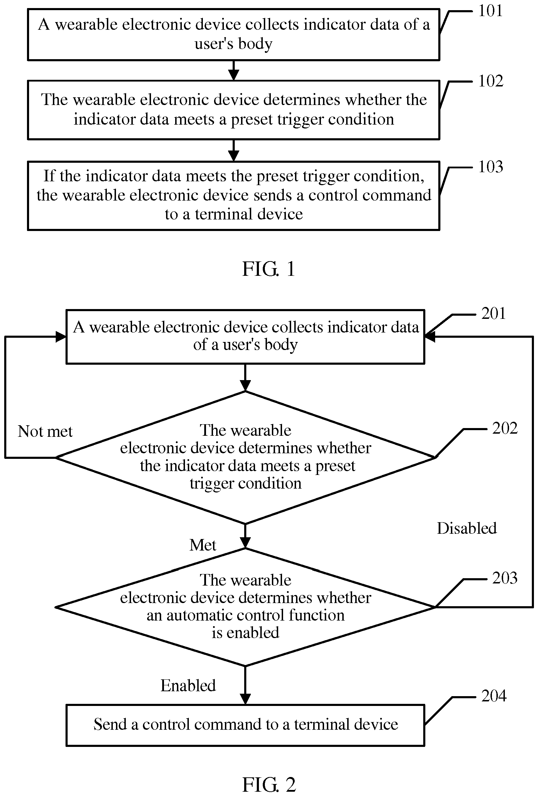

Referring to FIG. 1, an embodiment of a method for controlling a terminal device in the embodiments of the present invention includes the following steps:
101. A wearable electronic device collects indicator data of a user's body.

After starting working, the wearable electronic device collects the indicator data of the user's body.

102. The wearable electronic device determines whether the indicator data meets a preset trigger condition.

After collecting the indicator data of the user's body, the wearable electronic device determines whether the indicator data meets the preset trigger condition.

103. If the indicator data meets the preset trigger condition, the wearable electronic device sends a control command to the terminal device.

If the indicator data meets the preset trigger condition, the wearable electronic device sends the control command to the terminal device, so that the terminal device performs a corresponding operation according to the control command.

In this embodiment of the present invention, a wearable electronic device collects indicator data of a user's body, and when the indicator data meets a preset trigger condition, sends a control command, so that the terminal device performs a preset operation. In this way, the control command can be intelligently sent according to the indicator data collected by the wearable electronic device, so as to control another device, which reduces operations of the user, and greatly improves an affair-handling capability of the wearable electronic device.

In the foregoing embodiment, before sending the control command, the wearable electronic device may first determine whether an automatic control function is enabled. The following describes a method for controlling a terminal device in the embodiments of the present invention. Referring to FIG. 2, another embodiment of the method for controlling a terminal device in the embodiments includes the following steps:
201. A wearable electronic device collects indicator data of a user's body.

After starting working, the wearable electronic device collects the indicator data of the user's body.

The wearable electronic device represents a computer device that can be worn and controlled by a user and that keeps running and interworking. The wearable electronic device has many types of external forms, including a bracelet, a watch, glasses, even a body-implanted device, and other various body-combined intelligent devices, which are not limited herein.

It may be understood that there are many methods for triggering the wearable electronic device to start working. For example, the wearable electronic device may be triggered to start working by using a physical switch, touch control, or timing control, or collection may be started after the user chooses to activate a specific function, which is not limited herein.

The wearable electronic device collects the indicator data of the user's body in many manners. For example, the wearable electronic device may acquire a shell temperature parameter of the user by using a temperature sensor in the wearable electronic device, or may acquire a pulse parameter of the user by using a vibration sensor in the wearable electronic device, or may acquire a blood pressure parameter of the user by using a pressure sensor in the wearable electronic device, or may acquire a respiration parameter of the user by using a flow sensor in the wearable electronic device, or may acquire a blood glucose parameter of the user by using a heat flux sensor in the wearable electronic device, or may acquire an emotion parameter of the user by using a bioelectricity sensor in the wearable electronic device, or may acquire a temperature and humidity parameter by using a temperature and humidity sensor in the wearable electronic device, or may acquire an altitude by using a barometric pressure sensor in the wearable electronic device, or may acquire noise intensity by using a sound sensor in the wearable electronic device, or may acquire an acceleration parameter by using an acceleration sensor in the wearable electronic device, or may acquire a direction parameter by using a gyroscope in the wearable electronic device, or may acquire a motion parameter by using a camera in the wearable electronic device, or may acquire a time parameter by using a timer in the wearable electronic device, which is not limited herein.

202. The wearable electronic device determines whether the indicator data meets a preset trigger condition.

After collecting the indicator data of the user's body, the wearable electronic device determines whether the indicator data meets the preset trigger condition.

It should be noted that the preset trigger condition may be preset when the wearable electronic device leaves a factory, or may be customized by the user, which is not limited herein.

If the indicator data meets the preset trigger condition, step 203 is performed; or
if the indicator data does not meet the preset trigger condition, step 201 is performed.

In actual application, when the indicator data does not meet the preset trigger condition, it may also be set that when a quantity of times of failures in meeting the trigger condition reach a preset quantity of times within a preset time, the wearable electronic device suspends working, which is not limited herein.

203. The wearable electronic device determines whether an automatic control function is enabled.

When the indicator data meets the preset trigger condition, the wearable electronic device may determine whether the automatic control function is enabled.

If the automatic control function is enabled, step 204 is performed; or
if the automatic control function is not enabled, step 201 is performed.

In actual application, when determining that the automatic control function is not enabled, the wearable electronic device may perform step 203 to continue to detect whether the automatic control function is enabled, or may directly suspend working, which is not limited herein.

It should be noted that, according to a requirement of actual application, the wearable electronic device may first determine whether the automatic control function is enabled; if the automatic control function is enabled, the wearable electronic device further determines whether the indicator data meets the preset trigger condition, and if the indicator data meets the preset trigger condition, performs step 204, which is not limited herein.

204. Send a control command to the terminal device.

If the wearable electronic device determines that the automatic control function is enabled, the wearable electronic device sends the control command to the terminal device, so that the terminal device performs a corresponding operation according to the control command.

The terminal device may be a mobile phone, a tablet computer, or may be an intelligent switch, a router, or may further be another intelligent device, which is not limited herein.

It may be understood that, the control command corresponding to the terminal device may be preset by the wearable electronic device, or may be customized by the user, which is not limited herein.

In this embodiment of the present invention, before sending a control command, a wearable electronic device first determines whether an automatic control function is enabled, and when the automatic control function is enabled, sends the control command. In this way, execution of a function by the wearable electronic device can better meet an actual requirement of a user, which improves a degree of user experience.

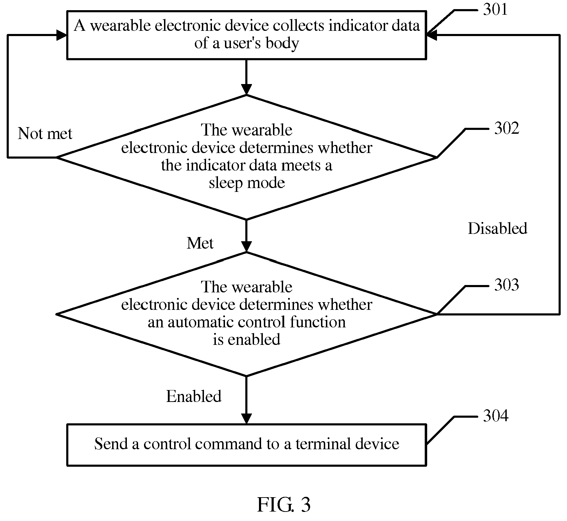

In the foregoing embodiment, the wearable electronic device determines whether the indicator data meets the preset trigger condition. In actual application, the wearable electronic device may determine whether the indicator data meets a sleep mode, and if the indicator data meets the sleep mode, it is determined that the indicator data meets the preset trigger condition. The following specifically describes a method for controlling a terminal device in the embodiments of the present invention. Referring to FIG. 3, another embodiment of the method for controlling a terminal device in the embodiments of the present invention includes the following steps:
301. A wearable electronic device collects indicator data of a user's body.

After starting working, the wearable electronic device collects the indicator data of the user's body.

It may be understood that there are many methods for triggering the wearable electronic device to start working. For example, the wearable electronic device may be triggered to start working by using a physical switch, touch control, or timing control, or collection may be started after the user chooses to activate a specific function, which is not limited herein.

The wearable electronic device collects the indicator data of the user's body in many manners. For example, the wearable electronic device may acquire a shell temperature parameter of the user by using a temperature sensor in the wearable electronic device, or may acquire a pulse parameter of the user by using a vibration sensor in the wearable electronic device, or may acquire a blood pressure parameter of the user by using a pressure sensor in the wearable electronic device, or may acquire a respiration parameter of the user by using a flow sensor in the wearable electronic device, or may acquire a blood glucose parameter of the user by using a heat flux sensor in the wearable electronic device, or may acquire an emotion parameter of the user by using a bioelectricity sensor in the wearable electronic device, or may acquire a temperature and humidity parameter by using a temperature and humidity sensor in the wearable electronic device, or may acquire an altitude by using a barometric pressure sensor in the wearable electronic device, or may acquire noise intensity by using a sound sensor in the wearable electronic device, or may acquire an acceleration parameter by using an acceleration sensor in the wearable electronic device, or may acquire a direction parameter by using a gyroscope in the wearable electronic device, or may acquire a motion parameter by using a camera in the wearable electronic device, and or may acquire a time parameter by using a timer in the wearable electronic device, which is not limited herein.

302. The wearable electronic device determines whether the indicator data meets a sleep mode.

After collecting the indicator data of the user's body, the wearable electronic device determines whether the indicator data meets the sleep mode, where the sleep mode is used to indicate that the user is in a sleep state.

If the indicator data meets the sleep mode, step 303 is performed; or
if the indicator data does not meet the sleep mode, step 301 is performed.

The determining whether the indicator data meets the sleep mode may be that: If a quantity of times of user motions is less than a preset quantity of times in a unit time, then the indicator data meets the sleep mode; if a range of a user motion is less than a preset range, then the indicator data meets the sleep mode; or if a pulse of the user is less than a preset value, then the indicator data meets the sleep mode. Further, a combination of various other types of indicator data may be used to indicate the sleep mode, which is not limited herein.

In actual application, when the indicator data does not meet the sleep mode, it may also be set that when a quantity of times of failures in meeting the sleep mode reach a preset quantity of times within a preset time, the wearable electronic device suspends working, which is not limited herein.

303. The wearable electronic device determines whether an automatic control function is enabled.

When the indicator data meets the sleep mode, the wearable electronic device may determine whether the automatic control function is enabled.

If the automatic control function is enabled, step 304 is performed; or
if the automatic control function is not enabled, step 301 performed.

In actual application, when determining that the automatic control function is not enabled, the wearable electronic device may perform step 303 to continue to detect whether the automatic control function is enabled, or may directly suspend working, which is not limited herein.

It should be noted that, according to a requirement of actual application, the wearable electronic device may first determine whether the automatic control function is enabled; if the automatic control function is enabled, the wearable electronic device further determines whether the indicator data meets a preset trigger condition, and if the indicator data meets the preset trigger condition, performs step 304, which is not limited herein.

304. Send a control command to the terminal device.

If the wearable electronic device determines that the automatic control function is enabled, the wearable electronic device sends the control command to the terminal device, so that the terminal device performs a corresponding operation according to the control command.

In this case, the user is in the sleep mode. To save resources and avoid disturbing the user's rest, the user may set: when the user is in the sleep mode, the wearable electronic device may send a television turn-off command or a light turn-off command to an intelligent switch, may also send a WIFI-off command to a router, and may also send an alarm clock start command to an electronic alarm clock. For example, after receiving a control command, sent by a wearable electronic device, for starting an alarm clock, the electronic alarm clock may start the alarm clock that has a predetermined moment set, and if the user is still in the sleep mode at the predetermined moment, the electronic alarm clock may ring to alarm the user, which is not limited herein.

In actual application, after receiving the control command, the terminal device may directly execute the control command, or may first determine whether there is an additional condition, and then perform the control command after the additional condition is met, which is not limited herein.

In this embodiment of the present invention, a wearable electronic device determines whether indicator data conforms to a sleep mode, and if the indicator data conforms to the sleep mode, a subsequent operation is performed. In this way, when a user falls asleep, the wearable electronic device may intelligently control a terminal device, which saves the user's time, reduces operations of the user, and improves a comfort degree of the user.

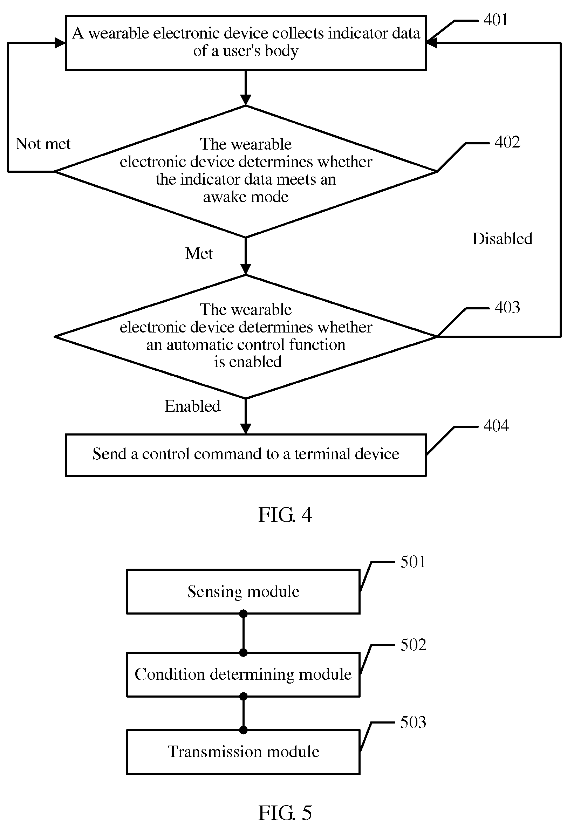

In the foregoing embodiment, the wearable electronic device determines whether the indicator data meets the sleep mode. In actual application, the wearable electronic device may also determine whether the indicator data meets an awake mode, and if the indicator data meets the awake mode, it is determined that the indicator data meets the preset trigger condition. The following specifically describes a method for controlling a terminal device in the embodiments of the present invention. Referring to FIG. 4, another embodiment of the method for controlling a terminal device in the embodiments of the present invention includes the following steps:
401. A wearable electronic device collects indicator data of a user's body.

After starting working, the wearable electronic device collects the indicator data of the user's body.

It may be understood that there are many methods for triggering the wearable electronic device to start working. For example, the wearable electronic device may be triggered to start working by using a physical switch, touch control, or timing control, or collection may be started after the user chooses to activate a specific function, which is not limited herein.

The wearable electronic device collects the indicator data of the user's body in many manners. For example, the wearable electronic device may acquire a shell temperature parameter of the user by using a temperature sensor in the wearable electronic device, or may acquire a pulse parameter of the user by using a vibration sensor in the wearable electronic device, or may acquire a blood pressure parameter of the user by using a pressure sensor in the wearable electronic device, or may acquire a respiration parameter of the user by using a flow sensor in the wearable electronic device, or may acquire a blood glucose parameter of the user by using a heat flux sensor in the wearable electronic device, or may acquire an emotion parameter of the user by using a bioelectricity sensor in the wearable electronic device, or may acquire a temperature and humidity parameter by using a temperature and humidity sensor in the wearable electronic device, or may acquire an altitude by using a barometric pressure sensor in the wearable electronic device, or may acquire noise intensity by using a sound sensor in the wearable electronic device, or may acquire an acceleration parameter by using an acceleration sensor in the wearable electronic device, or may acquire a direction parameter by using a gyroscope in the wearable electronic device, or may acquire a motion parameter by using a camera in the wearable electronic device, and or may acquire a time parameter by using a timer in the wearable electronic device, which is not limited herein.

402. The wearable electronic device determines whether the indicator data meets an awake mode.

After collecting the indicator data of the user's body, the wearable electronic device determines whether the indicator data meets the awake mode, where the awake mode indicates that the user is in an awake state.

If the indicator data meets the awake mode, step 403 is performed; or
if the indicator data does not meet the awake mode, step 401 is performed.

The determining whether the indicator data meets the awake mode may be that: If a quantity of times of user motions is greater than or equal to a preset quantity of times in a unit time, then the indicator data meets the awake mode; if a range of a user motion is greater than or equal to a preset range, then the indicator data meets the awake mode; or if a pulse of the user is greater than or equal to a preset value, then the indicator data meets the awake mode. Further, a combination of various other types of indicator data may be used to indicate the awake mode, which is not limited herein.

In actual application, when the indicator data does not meet the awake mode, it may also be set that when a quantity of times of failures in meeting the awake mode reach a preset quantity of times within a preset time, the wearable electronic device suspends working, which is not limited herein.

403. The wearable electronic device determines whether an automatic control function is enabled.

When the indicator data meets the awake mode, the wearable electronic device may determine whether the automatic control function is enabled.

If the automatic control function is enabled, step 404 is performed; or
if the automatic control function is not enabled, step 401 is performed.

In actual application, when determining that the automatic control function is not enabled, the wearable electronic device may perform step 403 to continue to detect whether the automatic control function is enabled, or may directly suspend working, which is not limited herein.

It should be noted that, according to a requirement of actual application, the wearable electronic device may first determine whether the automatic control function is enabled; if the automatic control function is enabled, the wearable electronic device further determines whether the indicator data meets a preset trigger condition, and if the indicator data meets the preset trigger condition, performs step 404, which is not limited herein.

404. Send a control command to the terminal device.

If the wearable electronic device determines that the automatic control function is enabled, the wearable electronic device sends the control command to the terminal device, so that the terminal device performs a corresponding operation according to the control command.

In this case, the user is in the awake mode. To save the user's time, the wearable electronic device may control to automatically start some necessary devices. For example, the wearable electronic device may send a command for enabling a wireless network transmission technology (WIFI, Wireless fidelity) to a router, and may also respectively send a light turn-on command or music automatic playing command to an intelligent switch or a mobile phone. For example, when the wearable electronic device sends a control command for automatic music playing to the mobile phone, after receiving the control command, the mobile phone may start a music playing application and play a piece of music preset by the user, which is not limited herein.

In actual application, after receiving the control command, the terminal device may directly execute the control command, or may first determine whether there is an additional condition, and then perform the control command after the additional condition is met, which is not limited herein.

In this embodiment of the present invention, a wearable electronic device determines whether indicator data conforms to an awake mode, and if the indicator data conforms to the awake mode, a subsequent operation is performed. In this way, when a user awakes from sleep, the wearable electronic device may intelligently control a terminal device, which saves the user's time, reduces operations of the user, and improves a comfort degree of the user.

In actual application, except determining the foregoing sleep mode and awake mode, the wearable electronic device may further determine many other modes. For example, a sport mode which indicates that a user is doing sports, or a study mode which indicates that a user is studying, which is not limited herein. A specific processing manner for each different mode, and an indicator data value range that meets each mode may be preset by the wearable electronic device, or may be customized by a user, which is not limited herein.

For ease of understanding, in the following, a specific application scenario is used to describe in detail the method for controlling a device in the embodiments of the present invention.

At current time, when the trigger condition for starting the wearable electronic device is met, the wearable electronic device starts working;
a quantity of times, of user motions, collected by an actigraphy in the wearable electronic device within 10 minutes is 20;
the wearable electronic device determines that 20 is less than the preset quantity of times, which is 30, and the wearable electronic device determines that the indicator data conforms to the sleep mode;
the wearable electronic device determines that the automatic control function is enabled;
the wearable electronic device sends a WIFI off command to a router; and
after the router receives the WIFI off command, it is determined whether all preset wearable electronic devices enter the sleep mode, and if all the preset wearable electronic devices enter the sleep mode, the router disables WIFI.

The following describes a wearable device in the embodiments of the present invention. Referring to FIG. 5, an embodiment of the wearable electronic device in the embodiments of the present invention includes:
a sensing module 501, configured to collect indicator data of a user's body; where
the sensing module may be an actigraphy, a temperature sensor, a vibration sensor, a pressure sensor, a flow sensor, a heat flux sensor, a bioelectricity sensor, or a gyroscope, or may further be a combination of all or a part of these sensors, which is not limited herein;
a condition determining module 502, configured to determine whether the indicator data collected by the sensing module 501 meets a preset trigger condition; and
a transmission module 503, configured to: when the condition determining module 502 determines that the indicator data meets the preset trigger condition, send a control command to a terminal device.

In this embodiment of the present invention, a sensing module 501 collects indicator data of a user's body, and when a condition determining module 502 determines that the indicator data meets a preset trigger condition, a transmission module 503 sends a control command, so that a terminal device performs a preset operation. In this way, another device may be intelligently controlled by using the indicator data detected by the wearable electronic device, which reduces operations of a user, and greatly improves an affair-handling capability of the wearable electronic device.

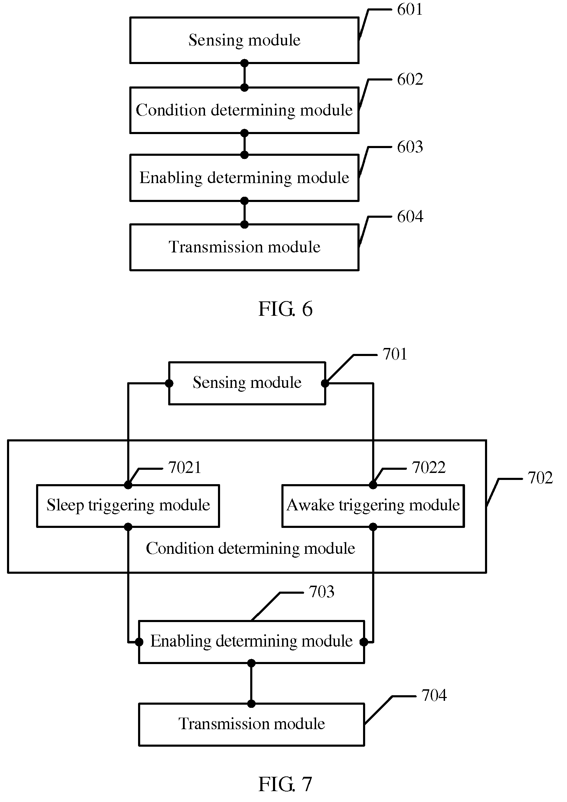

In the foregoing embodiment, before the transmission module 503 sends the control command, it may first be determined whether an automatic control function is enabled. The following describes a wearable electronic device in the embodiments of the present invention. Referring to FIG. 6, another embodiment of the wearable electronic device in the embodiments includes:
a sensing module 601, configured to collect indicator data of a user's body; where
the sensing module may be an actigraphy, a temperature sensor, a vibration sensor, a pressure sensor, a flow sensor, a heat flux sensor, a bioelectricity sensor, or a gyroscope, or may further be a combination of all or a part of these sensors, which is not limited herein;
a condition determining module 602, configured to determine whether the indicator data collected by the sensing module 601 meets a preset trigger condition; and
a transmission module 603, configured to: when the condition determining module 602 determines that the indicator data meets the preset trigger condition, send a control command to a terminal device.

In this embodiment, the wearable electronic device further includes:
an enabling determining module 604, configured to: when the condition determining module 602 determines that the indicator data meets the preset trigger condition, determine whether an automatic control function is enabled; where
the transmission module 603 is specifically configured to: when the enabling determining module 604 determines that the automatic control function is enabled, send the control command to the terminal device, so that the terminal device performs a corresponding operation according to the control command.

In this embodiment of the present invention, before a transmission module 603 sends a control command, an enabling determining module 604 first determines whether an automatic control function is enabled, and when the automatic control function is enabled, sends the control command. In this way, execution of a function by the wearable electronic device can better meet an actual requirement of a user, which improves a degree of user experience.

In the foregoing embodiment, the condition determining module 602 determines whether the indicator data meets the preset trigger condition. In actual application, it may be determined whether the indicator data meets a sleep mode or an awake mode. If the indicator data meets the sleep mode or the awake mode, it is determined that the indicator data meets the preset trigger condition. The following specifically describes a wearable electronic device in the embodiments of the present invention. Referring to FIG. 7, another embodiment of the wearable electronic device in the embodiments of the present invention includes:
a sensing module 701, configured to collect indicator data of a user's body; where
the sensing module may be an actigraphy, a temperature sensor, a vibration sensor, a pressure sensor, a flow sensor, a heat flux sensor, a bioelectricity sensor, or a gyroscope, or may further be a combination of all or a part of these sensors, which is not limited herein;
a condition determining module 702, configured to determine whether the indicator data collected by the sensing module 701 meets a preset trigger condition; and
a transmission module 703, configured to: when the condition determining module 702 determines that the indicator data meets the preset trigger condition, send a control command to a terminal device, so that the terminal device performs a corresponding operation according to the control command.

The wearable electronic device further includes:
an enabling determining module 704, configured to: when the condition determining module 702 determines that the indicator data meets the preset trigger condition, determine whether an automatic control function is enabled; and
the transmission module 703 is specifically configured to: when the enabling determining module 704 determines that the automatic control function is enabled, send the control command to the terminal device, so that the terminal device performs a corresponding operation according to the control command.

In this embodiment, the condition determining module 702 specifically includes:
a sleep triggering unit 7021, configured to determine whether the indicator data collected by the sensing module 701 conforms to a sleep mode, where if the indicator data conforms to the sleep mode, it is determined that the indicator data meets the preset trigger condition, where the sleep mode is used to indicate that the user is in a sleep state; and
an awake triggering unit 7022, configured to determine whether the indicator data collected by the sensing module 701 conforms to an awake mode, where if the indicator data conforms to the awake mode, it is determined that the indicator data meets the preset trigger condition, where the awake mode is used to indicate that the user is in an awake state.

In this embodiment of the present invention, a sleep triggering unit 7021 determines whether indicator data conforms to a sleep mode, and if the indicator data conforms to the sleep mode, a subsequent operation is performed. In this way, when a user falls asleep, the wearable electronic device may intelligently control a terminal device; and an awake triggering unit 7022 determines whether the indicator data conforms to an awake mode, and if the indicator data conforms to the awake mode, a subsequent operation is performed. In this way, when a user awakes from sleep, the wearable electronic device may intelligently control the terminal device, which saves the user's time, reduces operations of the user, and improves a comfort degree of the user.

For ease of understanding the foregoing embodiment, the following describes a process of interaction between the foregoing units of the wearable electronic device in a specific application scenario.

At current time, when the trigger condition for starting the wearable electronic device is met, the wearable electronic device starts working;
a quantity of times, of user micro-motions, collected by an actigraphy in the sensing module 701 within 10 minutes is 20;
the sleep triggering unit 7021 determines that 20 is less than the preset quantity of times, which is 30, and the wearable electronic device determines that the indicator data conforms to the sleep mode;
the enabling determining module 704 determines that the automatic control function is enabled;
the transmission module 703 sends a WIFI off command to a router;
after the router receives the WIFI off command, it is determined whether all preset wearable electronic devices enter the sleep mode, and if all the preset wearable electronic devices enter the sleep mode, the router disables WIFI.

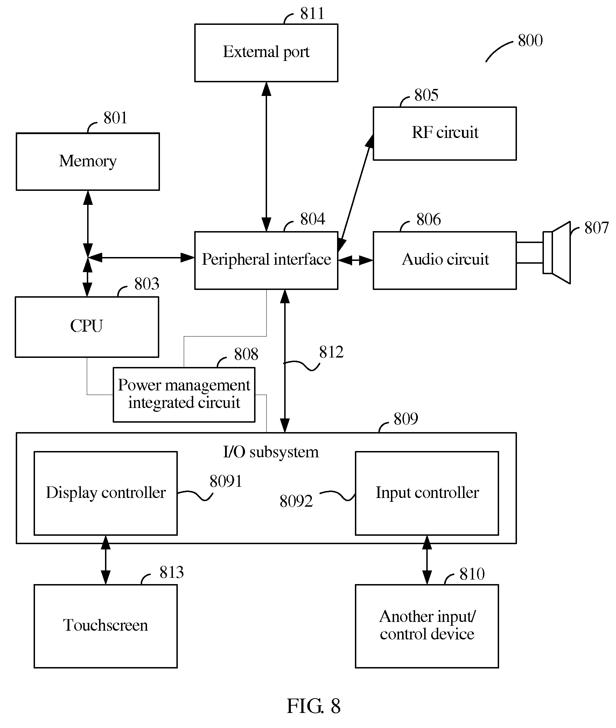

The foregoing describes the wearable electronic device in the embodiments of the present invention from a perspective of a unitized functional entity. The following specifically describes a data collection apparatus in an embodiment of the present invention from a perspective of hardware processing. Referring to FIG. 8, another embodiment of a wearable electronic device 800 in the embodiments of the present invention includes:
It should be understood that, the wearable electronic device 800 shown in the figure is merely an example of a wearable electronic device, and the wearable electronic device 800 may have components that are more than or less than components shown in the figure, may combine two or more components, or may have different component configurations. Various components shown in the figure may be implemented in hardware that includes one or more signals processing and/or application-specific integrated circuits, in software, or in a combination of hardware and software.

The wearable electronic device includes a memory 801, a central processing unit (Central Processing Unit, CPU for short) 803, a peripheral interface 804, an RF circuit 805, an audio circuit 806, a loudspeaker 807, a power management integrated circuit 808, an input/output (I/O) subsystem 809, another input/control device 810, and an external port 811. These components communicate by using one or more communications buses or signal cables 812.

It should be noted that, the wearable electronic device 800 provided in this embodiment is merely an example of a wearable electronic device, the wearable electronic device involved in this embodiment of the present invention may have components that are more than or less than components shown in FIG. 800, may combine two or more components, or may have different component configurations or settings. Each component may be implemented in hardware that includes one or more signals processing and/or application-specific integrated circuits, in software, or in a combination of hardware and software.

The following describes in detail the wearable electronic device that is for controlling a terminal device and is provided in this embodiment of the present invention.

Memory 801: The memory 801 may be accessed by the CPU 803, the peripheral interface 804 or the like, and the memory 801 may include a high-speed random access memory, or may further include a non-volatile memory, for example, one or more magnetic disk storage devices, a flash device, or another volatile solid-state storage device.

Peripheral interface 804: The peripheral interface may connect an input/output peripheral of a device to the CPU 803 and the memory 801.

I/O subsystem 809: The I/O subsystem 809 may connect an input/output peripheral of a device, for example, the touchscreen 813 (equivalent to a display in the foregoing embodiment), or the another input/control device 810, to the peripheral interface 804. The I/O subsystem 209 may include a display controller 8091, and one or more input controllers 8092 used for controlling the another input/control device 810. The one or more input controllers 8092 receive an electrical signal from the another input/control device 810 or send an electrical signal to the another input/control device 810. The another input/control device 810 may include a physical button (a press button, a rocker button, and the like), a dial, a slide switch, a joystick, and a click scroll wheel. It should be noted that the input controller 2092 may be connected to any of the following: a keyboard, an infrared port, a USB interface, and an indication device such as a mouse.

Touchscreen 813: The touchscreen 813 is an input interface and an output interface between the wearable electronic device and a user, and displays visual output to the user, where the visual output may include graphics, text, an icon, a video, and the like.

The display controller 8091 in the I/O subsystem 809 receives an electrical signal from the touchscreen 813 or sends an electrical signal to the touchscreen 813. The touchscreen 813 detects a touch on the touchscreen, and the display controller 8091 converts the detected touch into interaction of a user interface object displayed on the touchscreen 813, that is, implements human-machine interaction, where the user interface object displayed on the touchscreen 813 may be an icon for running a game, an icon for linking to a corresponding network, and the like. It should be noted that, the device may further include an optical mouse, where the optical mouse is a touch sensitive surface that does not display visual output, or an extension of a touch sensitive surface that is formed by a touchscreen.

The RF circuit 805 is mainly configured to establish communication between a wearable electronic device and a wireless network, that is, terminal devices connected to the wireless network, which implements data receive and transmit between the wearable electronic device and the wireless network, for example, sending a control command. Specifically, the RF circuit 805 receives and sends an RF signal, where the RF signal is also referred to as an electromagnetic signal. The RF circuit 805 converts an electrical signal into an electromagnetic signal or converts an electromagnetic signal into an electrical signal, and communicates with a communications network and another device by using the electromagnetic signal. The RF circuit 805 may include a known circuit used for executing these functions, including but not limited to an antenna system, an RF transceiver, one or more amplifiers, a tuner, one or more oscillators, a digital signal processor, a CODEC chip set, a subscriber identity module (Subscriber Identity Module, SIM), and the like. The RF circuit may be a WIFI circuit, a Bluetooth circuit, or an infrared circuit, which is not limited herein.

The audio circuit 806 is mainly configured to: receive audio data from the peripheral interface 804, converts the audio data into an electrical signal, and sends the electrical signal to the loudspeaker 807.

The loudspeaker 807 is configured to restore an audio signal, received by the wearable electronic device from a wireless network by using the RF circuit 805, to voice and play the voice for a user.

The power management integrated circuit 808 is configured to supply power to and perform power management on hardware that is connected with the CPU 803, the I/O subsystem, and the peripheral interface.

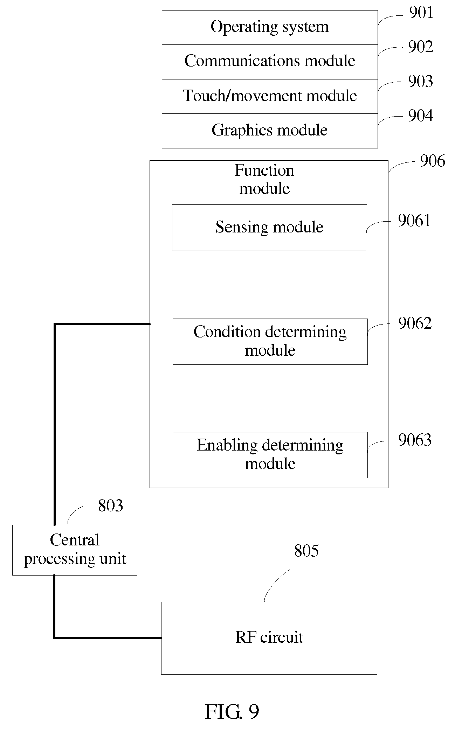

FIG. 9 is a structural diagram of an internal part of a wearable electronic device. In an embodiment of the present invention, software components stored in the memory 801 may include an operating system 901, a communication module 902, a touch/movement module 903, a graphics module 904, and a function module 906.

The operating system 901 (for example, Darwin, RTXC, LINUX, UNIX, OS X, WINDOWS, or an embedded operating system, such as VxWorks) includes various software components/drivers used for controlling and managing general system tasks (for example, memory management, storage device control, and power management), and facilitates communication between various hardware and software components.

The communication module 902 facilitates communication with another device by using one or more external ports 811, and further includes various software components used for processing data received by the RF circuit 805 and/or the external port 811.

The touch/movement module 903 may detect a touch on the touchscreen 813 (combined with the display controller 8091) and another touch sensitive device (for example, a touchpad, or a physical click scroll wheel). The touch/movement module 903 includes various software components that are used for performing various operations related to detecting a touch, where the operations include, for example, determining whether a touch occurs, determining whether the touch moves and tracing the movement on the touchscreen 813, and determining whether the touch is cut off (that is, whether the touch is stopped). Determining movement of a touch point may include determining a rate (an amplitude), a speed (an amplitude and a direction) and/or acceleration (change of an amplitude/a direction) of the touch point. These operations may be applied to a single touch (for example, a one-finger touch) or applied to multiple simultaneous touches (for example, a "multi-touch"/a multi-finger touch). In some embodiments, the touch/movement module 903 and the display controller 8091 further detect a touch on a touchpad.

The graphic module 904 includes various known software components used for displaying graphics on the touchscreen 813, including a component used for changing intensity of the displayed graphics, for example, after a command of the central processing unit 803 is received, graphical user interfaces of various types of software are displayed on the touchscreen 813.

The function module 906 is specifically at least one of the following modules:
a sensing module 9061, configured to collect indicator data of a user's body;
a condition determining module 9062, configured to determine whether the indicator data collected by the sensing module 9061 meets a preset trigger condition; and
an enabling determining module 9063, configured to: when the condition determining module 9062 determines that the indicator data meets the preset trigger condition, determine whether an automatic control function is enabled.

The central processing unit 803 identifies a data type of data in messages received and sent by the RF circuit 805, and the data is stored, according to a correspondence list, in a function module corresponding to the data type of the data, where the correspondence list is a correspondence list between a data type and a function module. The function module 906 may be specifically at least one module of the sensing module 9061, the condition determining module 9062, and the enabling determining module 9063. It may be understood that, in this embodiment of the present invention, a manner in which the central processing unit 803 identifies data in various formats may be performed as the manner in the foregoing embodiments, and details are not described herein again.

Optionally, after the central processing unit 803 determines whether the indicator data meets the preset trigger condition, if the indicator data meets the preset trigger condition, it may be further determined whether the automatic control function is enabled, and if it is determined that the automatic control function is enabled, a control command is sent to the terminal device. The central processing unit 803 may send data of various data types and data properties of the data to the function module 906 corresponding to a data type of the data. A specific technical solution has been described in detail in the second embodiment of the present invention, and details are not described herein again.

Specifically, when the central processing unit 803 determines whether the indicator data meets the preset trigger condition, whether the preset trigger condition is met may be determined by determining whether the indicator data meets a sleep mode or an awake mode. A specific technical solution has been described in detail in the third embodiment and the fourth embodiment of the present invention, and details are not described herein again.

In the foregoing embodiment, the CPU 203 may be specifically Pentium series processors or Itanium processors manufactured by Intel Corporation.

It should be noted that a person of ordinary skill in the art may understand that all or a part of the processes of the methods in the foregoing embodiments may be implemented by a computer program instructing relevant hardware. The program may be stored in a computer readable storage medium. When the program runs, the processes of the methods in the foregoing embodiments may be performed. The foregoing storage medium may be: a magnetic disk, an optical disc, a read-only memory, or a random access memory.

The foregoing describes in detail the method for controlling a terminal device by a wearable electronic device, and the device that are provided in the present invention. Specific embodiments are applied to illustrate principles and implementation manners of the present invention; and the foregoing embodiments are merely for ease of understanding of the method and core ideas of the present invention. In addition, for a person of ordinary skill in the art, on the basis of the idea of the present invention, a modification may be made to a specific implementation manner and an application range. In conclusion, the content of this specification shall not be construed as a limitation to the present invention. The terms used in the embodiments of the present invention are merely for the purpose of illustrating specific embodiments, and are not intended to limit the present invention. The terms "a", "said" and "the" of singular forms used in the embodiments and the appended claims of the present invention are also intended to include plural forms, unless otherwise specified in the context clearly. It should also be understood that, the term "and/or" used herein indicates and includes any or all possible combinations of one or more associated listed items.

It may be clearly understood by a person skilled in the art that, for the purpose of convenient and brief description, for a detailed working process of the foregoing system, apparatus, and unit, reference may be made to a corresponding process in the foregoing method embodiments, and details are not described herein again.

In the several embodiments provided in the present application, it should be understood that the disclosed system, apparatus, and method may be implemented in other manners. For example, the described apparatus embodiment is merely exemplary. For example, the unit division is merely logical function division and may be other division in actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electronic, mechanical, or other forms.

The units described as separate parts may or may not be physically separate, and parts displayed as units may or may not be physical units, may be located in one position, or may be distributed on a plurality of network units. Some or all of the units may be selected according to actual needs to achieve the objectives of the solutions of the embodiments.

In addition, functional units in the embodiments of the present invention may be integrated into one processing unit, or each of the units may exist alone physically, or two or more units are integrated into one unit. The integrated unit may be implemented in a form of hardware, or may be implemented in a form of a software functional unit.

When the integrated unit is implemented in the form of a software functional unit and sold or used as an independent product, the integrated unit may be stored in a computer-readable storage medium. Based on such an understanding, the technical solutions of the present invention essentially, or the part contributing to the prior art, or all or some of the technical solutions may be implemented in the form of a software product. The software product is stored in a storage medium and includes several instructions for instructing a computer device (which may be a personal computer, a server, or a network device) to perform all or some of the steps of the methods described in the embodiments of the present invention. The foregoing storage medium includes: any medium that can store program code, such as a USB flash drive, a removable hard disk, a read-only memory (ROM, Read-Only Memory), a random access memory (RAM, Random Access Memory), a magnetic disk, or an optical disc.

The foregoing embodiments are merely intended for describing the technical solutions of the present invention, but not for limiting the present invention. Although the present invention is described in detail with reference to the foregoing embodiments, persons of ordinary skill in the art should understand that they may still make modifications to the technical solutions described in the foregoing embodiments or make equivalent replacements to some technical features thereof, without departing from the spirit and scope of the technical solutions of the embodiments of the present invention.

## Claims

1. A method for controlling a terminal device, comprising:
collecting, by a wearable electronic device, indicator data of a user's body;
determining, by the wearable electronic device, whether the indicator data meets a preset trigger condition; and
if the indicator data meets the preset trigger condition, sending, by the wearable electronic device, a control command to the terminal device.

2. The method according to claim 1, before the sending, by the wearable electronic device, a control command to the terminal device, comprising:
determining, by the wearable electronic device, whether an automatic control function is enabled; and
if it is determined that the automatic control function is enabled, performing, by the wearable electronic device, the step of sending, by the wearable electronic device, a control command to the terminal device.

3. The method according to claim 1 or 2, wherein the determining, by the wearable electronic device, whether the indicator data meets a preset trigger condition comprises:
determining, by the wearable electronic device, whether the indicator data conforms to a sleep mode, wherein if the indicator data conforms to the sleep mode, it is determined that the indicator data meets the preset trigger condition, wherein the sleep mode is used to indicate that the user is in a sleep state; and/or
determining, by the wearable electronic device, whether the indicator data conforms to an awake mode, wherein if the indicator data conforms to the awake mode, it is determined that the indicator data meets the preset trigger condition, wherein the awake mode is used to indicate that the user is in an awake state.

4. The method according to claim 3, wherein:
the indicator data comprises:
a quantity, of times of user motions, recorded by an actigraphy in the wearable electronic device;
the determining, by the wearable electronic device, whether the indicator data conforms to a sleep mode comprises:
if the quantity of times of user motions is less than a preset quantity of times, determining, by the wearable electronic device, that the indicator data conforms to the sleep mode; and
the determining, by the wearable electronic device, whether the indicator data conforms to an awake mode comprises:
if the quantity of times of user motions is greater than or equal to the preset quantity of times, determining, by the wearable electronic device, that the indicator data conforms to the awake mode.

5. The method according to claim 3, wherein:
the indicator data comprises:
a range, of a user motion, recorded by an actigraphy in the wearable electronic device;
the determining, by the wearable electronic device, whether the indicator data conforms to a sleep mode comprises:
if the range of the user motion is less than a preset range, determining, by the wearable electronic device, that the indicator data conforms to the sleep mode; and
the determining, by the wearable electronic device, whether the indicator data conforms to an awake mode comprises:
if the range of the user motion is greater than or equal to the preset range, determining, by the wearable electronic device, that the indicator data conforms to the awake mode.

6. A wearable electronic device, comprising:
a sensing module, configured to collect indicator data of a user's body;
a condition determining module, configured to determine whether the indicator data collected by the sensing module meets a preset trigger condition; and
a transmission module, configured to: when the condition determining module determines that the indicator data meets the preset trigger condition, send a control command to a terminal device.

7. The device according to claim 6, wherein
the device further comprises:
an enabling determining module, configured to: when the condition determining module determines that the indicator data meets the preset trigger condition, determine whether an automatic control function is enabled; and
the transmission module is specifically configured to: when the enabling determining module determines that the automatic control function is enabled, send the control command to the terminal device.

8. The device according to claim 7, wherein the condition determining module specifically comprises:
a sleep triggering unit, configured to determine whether the indicator data collected by the sensing module conforms to a sleep mode, wherein if the indicator data conforms to the sleep mode, it is determined that the indicator data meets the preset trigger condition, wherein the sleep mode is used to indicate that the user is in a sleep state;
and/or
an awake triggering unit, configured to determine whether the indicator data collected by the sensing module conforms to an awake mode, wherein if the indicator data conforms to the awake mode, it is determined that the indicator data meets the preset trigger condition, wherein the awake mode is used to indicate that the user is in an awake state.

9. A wearable electronic device, comprising:
a memory, a central processing unit, a peripheral interface, an RF circuit, a power management integrated circuit, an input/output subsystem, another input/control device, an external port, and a communications bus; wherein
the central processing unit performs the following operations:
collecting indicator data of a user's body;
determining whether the indicator data meets a preset trigger condition; and
when the indicator data meets the preset trigger condition, sending a control command to a terminal device.

10. The apparatus according to claim 9, wherein the central processing unit specifically performs the following operations:
when the indicator data meets the preset trigger condition, determining whether an automatic control function is enabled; and
when it is determined that the automatic control function is enabled, triggering to perform the step of sending a control command to a terminal device.

11. The apparatus according to claim 10, wherein the central processing unit specifically performs the following operations:
determining whether the indicator data conforms to a sleep mode, wherein if the indicator data conforms to the sleep mode, it is determined that the indicator data meets the preset trigger condition, wherein the sleep mode is used to indicate that the user is in a sleep state;
and/or
determining whether the indicator data conforms to an awake mode, wherein if the indicator data conforms to the awake mode, it is determined that the indicator data meets the preset trigger condition, wherein the awake mode is used to indicate that the user is in an awake state.
